# EUROPÄISCHE PATENTSCHRIFT

(11) **EP 0 514 652 B1**
(45) Veröffentlichungstag und Bekanntmachung des Hinweises auf die Patenterteilung: **06.09.1995**
(21) Anmeldenummer: 92105576.0
(22) Anmeldetag: 01.04.1992
(51) Int. Cl.: C07C 43/315, C07C 41/54, C11D 1/72

(54) **Alkandiolbisacetale**
Alcanediolbisacetals
Alkanediolbisacetal

(30) Priorität: 23.04.1991 DE 4113163
(43) Veröffentlichungstag der Anmeldung: 25.11.1992
(73) Patentinhaber: BASF Aktiengesellschaft, 67063 Ludwigshafen (DE)
(72) Erfinder: Baur, Richard, Dr., W-6704 Mutterstadt (DE); Guembel, Helmut, Dr., W-6700 Ludwigshafen (DE); Oftring, Alfred, Dr., W-6702 Bad Duerkheim (DE); Perner, Johannes, Dr., W-6730 Neustadt (DE); Wolf, Gerhard, Dr., W-6800 Mannheim 24 (DE)

(56) Entgegenhaltungen:
- DE-A- 2 252 186
- FR-A- 2 204 683
- US-A- 2 905 719

## Beschreibung

Die Vorliegende Erfindung betrifft neue Alkandiolbisacetale der allgemeinen Formel I 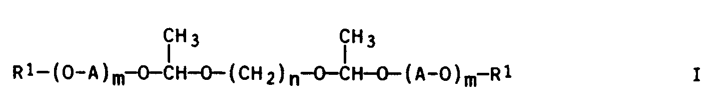
in der die Variablen
- R¹: Alkyl- oder Alkenylgruppen mit 6 bis 30 C-Atomen bedeuten,
- A: 1,2-Alkylengruppen mit 2 bis 4 C-Atomen bezeichnen und
- m: für eine Zahl von 0 bis 50 stehen,
wobei die Variablen R¹, A und m jeweils gleich oder verschieden sein können, und
- n: eine Zahl von 2 bis 20 bedeutet.

Weiterhin betrifft die Erfindung ein Verfahren zur Herstellung dieser Alkandiolbisacetale, ihre Verwendung als oberflächenaktive Substanzen für industrielle Anwendungszwecke, insbesondere in Wasch- und Reinigungsmitteln, sowie die Verbindungen I enthaltende Wasch-und Reinigungsmittel.

Wasch- und Reinigungsprozesse in Industrie, Gewerbebetrieben und Haushalt verlangen heutzutage immer mehr nach oberflächenaktiven Substanzen, welche sich vor allem durch gute Alkalistabilität, Schaumarmut und effektive Schaumdämpfung auszeichnen, insbesondere bei maschinell ablaufenden Reinigungsprozessen.

Auf der Suche nach Verbindungen, die diesen Anforderungen gerecht werden, wurden bereits Alkoxylate mit einer Monoacetalstruktur vorgeschlagen, beispielsweise in der DE-A 22 52 186 Verbindungen des Typs 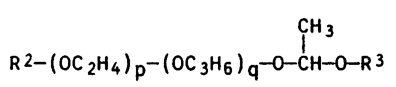
wobei R² einen langkettigen Alkylrest oder einen Alkylarylrest bezeichnet, R³ einen kürzerkettigen Alkylrest bedeutet und p und q für Zahlen von 1 bis 30 bzw. 5 bis 50 stehen. Derartige Monoacetale erweisen sich aber in ihren wasch- und reinigungstechnischen Eigenschaften immer noch als verbesserungsbedürftig.

Somit lag der vorliegenden Erfindung die Aufgabe zugrunde, den geschilderten Mängeln des Standes der Technik abzuhelfen.

Demgemäß wurden die eingangs definierten Alkandiolbisacetale I gefunden.

Als geradkettige oder verzweigte Alkyl- und Alkenylgruppen R¹ seien beispielsweise genannt: n-Hexyl, n-Heptyl, n-Octyl, 2-Ethylhexyl, n-Nonyl, iso-Nonyl, n-Decyl, iso-Decyl, n-Undecyl, n-Dodecyl, n-Tridecyl, iso-Tridecyl, n-Tetradecyl, n-Pentadecyl, n-Hexadecyl, n-Heptadecyl, n-Octadecyl, n-Eicosyl, Oleyl, Linolyl und Linolenyl. Die Reste R¹ sind vorzugsweise geradkettig oder nur in geringem Umfang verzweigt, d.h. sie enthalten maximal 3 Methyl- oder Ethylseitenketten.

Je nach der Herkunft des bei der Synthese der Verbindungen I eingesetzten Alkohols handels es sich bei R¹ um Reste von natürlich vorkommenden Fettalkoholen oder vorzugsweise von synthetisch hergestellten Oxo- oder Ziegler-Alkoholen. Beispiele für gut einsetzbare nach der Oxosynthese hergestellte Alkohole sind C₁₀-, C₁₃- und C₁₅-Alkohole sowie C₉/C₁₁-, C₁₀/C₁₂-, C₁₂/C₁₄-, C₁₃/C₁₅- und C₁₆/C₁₈-Alkanolgemische. Beispiele für gut einsetzbare nach der Ziegler-Synthese hergestellte Alkohole sind C₈/C₁₀-, C₁₀/C₁₂-, C₁₂/C₁₄-, C₁₂/₁₆-, C₁₆/C₁₈- und C₁₆/C₂₀-Alkanolgemische.

Da die bei der Synthese der Verbindungen I eingesetzten Alkohole in der Regel statistische Homologen- und auch Isomerengemische darstellen, ist es zweckmäßig, bei den Resten R¹ von einer durchschnittlichen Anzahl der C-Atome zu sprechen.

Bevorzugt werden Verbindungen I, bei denen R¹ Alkyl- oder Alkenylgruppen mit 9 bis 18 C-Atomen, insbesondere mit 10 bis 16 C-Atomen, bedeuten. Besonders vorteilhaft sind solche Reste R¹, die auf die C₁₀-Fraktion, die C₁₃-Fraktion, den C₁₀/C₁₂-, den C₁₂/C₁₄-, den C₁₃/C₁₅- oder den C₁₆/C₁₈-Schnitt eines nach der Oxosynthese erhaltenen Alkohols zurückgeführt werden können.

Die 1,2-Alkylengruppen A bezeichnen insbesondere die Ethylen-, daneben aber auch die Propylen-, 1,2-Butylen- und 2,3-Butylengruppe. Dabei kann jede Gruppe A auch ein statistisches Gemisch aus mehreren der genannten 1,2-Alkylengruppen oder eine aus bis zu drei einheitlichen Blöcken dieser Alkylengruppen aufgebaute Gruppe bezeichnen; bevorzugt werden jedoch 1,2-Alkylengruppen A, die nur eine einzige Baueinheit enthalten.

Die Alkoxylierungsgrade m liegen zwischen 0 und 50, vorzugsweise 2 und 15, insbesondere 3 und 12. Die Werte für m stellen üblicherweise Durchschnittswerte dar.

Die Variablen R¹, A und m sind vorzugsweise jeweils gleich, so daß es sich bei den Verbindungen I um symmetrische Moleküle handelt.

Die Anzahl der Methylen-Brückenglieder n beträgt 2 bis 20, vorzugsweise 2 bis 10, insbesondere 2 bis 6; ganz besonders bevorzugt wird der Wert 4.

Zweckmäßigerweise stellt man die Alkandiolbisacetale 1 dadurch her, daß man Alkandiolbisvinylether der allgemeinen Formel II

H₂C=CH-O-(CH₂)ₙ-O-CH=CH₂ II

in der n die obengenannte Bedeutung hat, mit alkoxylierten Alkoholen der allgemeinen Formel III

R¹-(O-A)ₘ-OH III

in der R¹, A und m die obengenannten Bedeutungen haben, oder einem Gemisch solcher Alkohole bei Temperaturen von 0 bis 100°C in Gegenwart katalytischer Mengen Säure umsetzt.

Die Umsetzung erfolgt vorzugsweise bei 10 bis 60°C, insbesondere bei 20 bis 40°C, und in der Regel bei Normaldruck. Ein zusätzliches inertes Lösungs- oder Verdünnungsmittel ist normalerweise nicht notwendig, kann aber im Bedarfsfall, beispielsweise bei Viskositätsproblemen, zugesetzt werden. Wesentlich für eine saubere Umsetzung ohne Nebenprodukte ist die weitgehende Abwesenheit von Wasser und niederen Alkoholen.

Die Umsetzung der Bisvinylether II mit den Alkoxylaten III wird unter saurer Katalyse durchgeführt. Als Katalysator eignen sich sowohl Lewissäuren, z.B. BF₃, AlCl₃, ZnCl₂ oder TiCl₄, als auch Mineralsäuren, z.B. HCl, H₂SO₄, H₃PO₄, H₃PO₃ oder HClO₄. Ebenfalls gut geeignet sind organische Carbon- und Sulfonsäuren, z.B. Methansulfonsäure, p-Toluolsulfonsäure, Oxalsäure, Ameisensäure, Essigsäure, Propionsäure oder Dodecylbenzolsulfonsäure. Besonders vorteilhaft läßt sich p-Toluolsulfonsäure als Katalysator verwenden.

Der saure Katalysator wird in den hierbei üblichen Mengen zugesetzt, also normalerweise in einer Menge von etwa 0,1 bis 5 mol-%, bezogen auf eingesetztes Alkoxylat III. Eine Neutralisation des sauren Katalysators nach erfolgter Umsetzung kann mit anorganischen Basen, z.B. NaOH, KOH, K₂CO₃, Na₂CO₃, oder organischen Basen, z.B. Trimethylamin, Triethylamin, Dimethylcyclohexylamin, Pyridin oder dem Umsetzungsprodukt von Ethylendiamin mit 4 mol Propylenoxid, durchgeführt werden.

Der Verlauf der Umsetzung läßt sich gut mittels IR-Spektroskopie verfolgen, wobei die Abnahme bzw. das endgültige Verschwinden der O-H-Streckschwingung der eingesetzten alkoxylierten Alkohole III als Kriterium für einen vollständigen Reaktionsablauf genommen wird.

Die alkoxylierten Alkohole III können in bekannter Weise durch Alkoxylierung der entsprechenden, oben genannten Fettalkohole, Oxoalkohole oder Ziegler-Alkohole hergestellt werden.

Die Alkandiolbisvinylether II lassen sich ebenfalls in bekannter Weise aus den entsprechenden Diolen durch Addition an Acetylen herstellen.

Die erfindungsgemäßen Alkandiolbisacetale I eignen sich generell als oberflächenaktive Substanzen für industrielle Anwendungszwecke und weisen somit eine Vielzahl von technischen Anwendungsmöglichkeiten auf. Mögliche Einsatzgebiete sind z.B. die Wasch- und Reinigungsmittelindustrie, die Galvanotechnik, die photographische Industrie, die Textilindustrie, die Papierindustrie, die Erdölförderung, die pharmazeutische Industrie, die kosmetische Industrie, die Nahrungsmittelindustrie und die Pflanzenernährung.

Die erfindungsgemäßen Verbindungen I sind insbesondere geeignet als oberflächenaktive Substanzen in Wasch- und Reinigungsmitteln für Industrie, Gaststättengewerbe und Haushalt, insbesondere für maschinell ablaufende Reinigungsprozesse in der Metall-, Papier-, Textil- oder Nahrungsmittelindustrie, beispielsweise für die industrielle Flaschenwäsche oder für die maschinelle Geschirreinigung.

Für die Reinigung von Flaschen in der Getränkeindustrie werden hochalkalische Reiniger eingesetzt. Das Alkali löst, neutralisiert bzw. verseift Getränkereste und Rückstände und führt den Etikettenleim in eine stark schäumende wasserlösliche Form über. Alle diese Prozesse laufen bei hoher Mechanik ab und begünstigen somit die ohnehin große Schaumneigung von Stärke und Zuckerabbauprodukten.

Eine andere Anwendung betrifft die industriellen Reinigungsprozesse in der Metallindustrie. Auch hier wird mit hohem Druck eine sehr gut netzende alkalische wäßrige Lösung als Reinigungsmedium zum Entfernen von Zieh- und Walzfetten bzw. carboxylgruppenhaltigen organischen Korrosionsinhibitoren eingesetzt. Hier sollen die erfindungsgemäßen Tenside nicht nur die Netzeigenschaften verbessern, sondern insbesondere zur Schaumdämpfung von z.B. anionischen Tensiden vom Typ der Alkylbenzolsulfonate bzw. anderer Sulfonsäuregruppen- und Carboxylgruppen-haltiger Tenside beitragen.

Weiterhin sind Gegenstand der vorliegenden Erfindung Wasch- und Reinigungsmittel, die neben den hierfür üblichen Bestandteilen 0,1 bis 50 Gew.-%, vorzugsweise 1 bis 30 Gew.-%, bezogen auf die Gesamtmenge der Zubereitung, eines oder mehrerer erfindungsgemäßer Alkandiolbisacetale I enthalten. Die Bestandteile und die Zusammensetzung derartiger Wasch- und Reinigungsmittel sind dem Fachmann bekannt und brauchen deshalb hier nicht näher erläutert zu werden.

Die erfindungsgemäßen Alkandiolbisacetale I sind alkalistabil und zeichnen sich durch ihre guten Anwendungseigenschaften wie effiziente Erniedrigung der Oberflächenspannung, gutes Netzvermögen, Schaumarmut und vor allem gute Schaumdämpfung bei gleichzeitiger biologischer Abbaubarkeit von in der Regel mindestens 80 % aus.

### Herstellungsbeispiele

### Beispiel 1

408,9 g (entsprechend 1,0 mol) eines mit 5 mol Ethylenoxid umgesetzten C₁₃-Oxoalkohols (OH-Zahl: 137,2 mg KOH/g) wurden mit 3,8 g (entsprechend 0,02 mol) p-Toluolsulfonsäuremonohydrat versetzt und 2 Stunden beim 100°C und ca. 10 mbar entwässert. Die Mischung wurde auf 20 bis 25°C abgekühlt und bei dieser Temperatur wurden 77,1 g (entsprechend 0,5 mol) Butan-1,4-diolbisvinylether innerhalb von 2 Stunden zugetropft. Man ließ 20 Minuten bei Raumtemperatur nachrühren und neutralisierte den Katalysator mit 2,5 g (entsprechend 0,02 mol) Dimethylcyclohexylamin. Nach Filtration erhielt man 472 g Produkt (entsprechend einer Ausbeute von 97 %) mit einer OH-Zahl von 2,9 mg KOH/g.

### Beispiele 2 bis 14

Analog Beispiel 1 wurden die in Tabelle 1 aufgeführten alkoxylierten Alkohole mit Butan-1,4-diolbisvinylether zu den Produkten 2 bis 14 umgesetzt.

**Tabelle 1**

| Alkandiolbisacetale aus alkoxylierten Alkoholen und Butan-1,4-diolbisvinylether | | | | |
|---|---|---|---|---|
| Beispiel Nr. | Alkohol | Ethoxylierungsgrad m | Produkt OH-Zahl [mg KOH/g] | Ausbeute [%] |
| 2 | C₁₃-Oxoalkohol | 3 | 3,1 | 95 |
| 3 | C₁₃-Oxoalkohol | 12 | 2,9 | 93 |
| 4 | C₁₃/C₁₅-Oxoalkohol | 3 | 5,8 | 97 |
| 5 | C₁₃/C₁₅-Oxoalkohol | 5 | 6,9 | 95 |
| 6 | C₁₃/C₁₅-Oxoalkohol | 7 | 3,3 | 94 |
| 7 | C₁₃/C₁₅-Oxoalkohol | 10 | 3,3 | 95 |
| 8 | C₁₀-Oxoalkohol | 3 | 6,3 | 97 |
| 9 | C₁₀-Oxoalkohol | 5 | 4,8 | 93 |
| 10 | C₁₀-Oxoalkohol | 7 | 3,6 | 94 |
| 11 | C₁₀-Oxoalkohol | 11 | 3,5 | 95 |
| 12 | C₁₀/C₁₂-Oxoalkohol | 6 | 8,0 | 96 |
| 13 | C₁₂/C₁₄-Oxoalkohol | 3 | 5,0 | 96 |
| 14 | C₁₆/C₁₈-Oxoalkohol | 9 | 5,0 | 97 |

### Anwendungstechnische Eigenschaften

Die Prüfung des Schaumvermögens erfolgte gemäß DIN 53 902 bei 40°C mit 2 g/l Prüfsubstanz. Dabei wurde das Schaumvolumen in ml 30 sec nach Beendigung der Schaumerzeugung bestimmt.

Zur weitern Charakterisierung wurde das Netzvermögen durch Tauchen eines Baumwollgewebes in die zu untersuchende Tensidlösung gemäß DIN 53 901 geprüft. Die Messung wurde mit 2 g/l Prüfsubstanz und 2 g/l Natriumcarbonat in destilliertem Wasser bei 20°C durchgeführt. Dabei wurde die Zeit in sec gemessen, nach der das Gewebe seinen durch die eingeschlossene Luft verursachten Auftrieb verliert und zu sinken beginnt. Je kürzer die Zeitspanne, desto besser ist das Netzvermögen.

Die Messung der Oberflächenspannung erfolgte gemäß DIN 53 914 bei 20°C mit 0,1 g/l Prüfsubstanz. Dabei wurde die Kraft in mN/m gemessen, welche notwendig ist, um einen horizontal aufgehängten Ring oder Bügel aus der Flüssigkeitsoberfläche herauszuziehen.

Das Schaumdämpfungsverhalten wurde den unterschiedlichen Anwendungen entsprechend einmal in der Geschirrspülmaschine unter Eiweißbelastung geprüft ("Ei-Test"), zum anderen wurde die schaumdämpfende Wirkung auf C₁₂/C₁₄-α-Olefinsulfonat in der dynamischen Schaumapparatur untersucht.

Beim sogenannten "Ei-Test" wurde durch magnetische Induktionsmessung in einem Geschirrspülautomaten (Miele Typ G 7735) mit Hilfe eines Zählwerks die Zahl der Umdrehungen eines Sprüharms bestimmt. Durch Schaumbildung, die besonders bei Anwesenheit von Proteinen (Eiweiß) auftritt, wird die Umdrehungszahl des Sprüharms vermindert. Die Umdrehungszahl stellt somit wegen der verringerten Rückstoßkraft ein Maß für die Tauglichkeit von Tensiden in Reinigungsgeräten mit hoher Mechanik dar. Die Testzeit betrug 12 Minuten, wobei die Umdrehungszahl pro Minute aus der Gesamtumdrehungszahl berechnet wurde. Der Waschvorgang wurde bei Raumtemperatur begonnen, nach etwa 10 Minuten betrug die Temperatur des Spülwassers 60°C.

Die Schaumdämpfung auf C₁₂/C₁₄-α-Olefinsulfonat in der dynamischen Schaumapparatur ist eine Labormethode, um das Schaumdämpfungsverhalten auf anionische Tenside zu untersuchen. Bei der Prüfapparatur handelt es sich um eine kontinuierlich arbeitende Umlaufapparatur. Hierbei wird der Schaumaufbau dadurch hervorgerufen, daß in einem temperierten, kalibrierten Rohr von 10 cm Durchmesser ein Prüfstrahl kontinuierlich mit konstantem Druck in die Vorlagelösung strömt, wobei gleichzeitig feinverteilte Luft in die Vorlagelösung geleitet wird. Wird der Prüflösung ein Schaumbooster in Form von C₁₂/C₁₄-α-Olefinsulfonat zugesetzt, so baut sich zeitabhängig eine produktspezifische Schaumhöhe auf, die nach 10 Minuten in cm gemessen wird. Die Schaumhöhe bezieht sich auf einem Einsatz von 1000 ppm Schaumbooster bei Zusatz von 40 ppm der Prüfsubstanz. Je geringer die Schaumhöhe, desto größer ist das Dämpfungspotential des Tensids.

Tabelle 2 zeigt die Ergebnisse der geschilderten anwendungstechnischen Prüfungen.

**Tabelle 2**

| Schaumvermögen, Netzvermögen, Oberflächenspannung und Schaumdämpfungsverhalten von Alkandiolbisacetalen | | | | | |
|---|---|---|---|---|---|
| Beispiel Nr. | Schaumvermögen [ml] | Netzvermögen [sec] | Oberflächenspannung [mN/m] | "Ei-Test" [U/min] | Schaumdämpfung auf C₁₂/C₁₄-α-Olefinsulfonat [cm] |
| 1 | 10 | > 300 | 28,7 | 102 | 50 |
| 2 | 0 | 67 | 28,6 | 110 | 19 |
| 3 | 80 | 63 | 30,4 | 92 | 72 |
| 4 | 10 | 95 | 29,0 | 109 | 26 |
| 5 | 10 | 65 | 29,0 | 104 | 45 |
| 6 | 40 | > 300 | 29,3 | 106 | 63 |
| 7 | 50 | 160 | 30,5 | 116 | 72 |
| 8 | 0 | 100 | 29,1 | 107 | 32 |
| 9 | 0 | 68 | 29,5 | 112 | 49 |
| 10 | 10 | 85 | 29,4 | 116 | 61 |
| 11 | 50 | 85 | 31,6 | 112 | 60 |
| 12 | 20 | 95 | 29,4 | 104 | 59 |
| 13 | 0 | > 300 | 29,2 | 105 | 47 |
| 14 | 0 | > 300 | 32,2 | 107 | 64 |

Aus den Ergebnissen der Tabelle 2 geht hervor, daß nach dem Schaumtest gemäß DIN 53 902 mit wenigen Ausnahmen alle untersuchten Produkte praktisch keinen Schaum bilden.

Der praxisnahe "Ei-Test" in der Geschirrspülmaschine zeigt, daß die schaumdämpfenden Eigenschaften auf Eiweißschaum hervorragend sind, denn bereits Werte über 80 U/min bedeuten eine ausgezeichnete Schaumdämpfung.

Die Werte für das Netzvermögen zeigen, daß abhängig vom Ethoxylierungsgrad auch Produkte mit hoher Schaumdämpfung ausgezeichnete Benetzungseffekte zeigen können.

Mit den Produkten gemäß Beispiel 2, 4 und 8 lassen sich Schaumdämpfungswerte, gemessen in der dynamischen Schaumapparatur, erreichen, die bisher nur mit biologisch nicht abbaubaren Tensiden erhalten wurden.

Mit den erfindungsgemäßen Alkandiolbisacetalen I konnten damit Produkte zur Verfügung gestellt werden, die je nach Struktur und Ethoxylierungsgrad neben Alkalistabilität und biologischer Abbaubarkeit ausgezeichnete Schaumdämpfung mit guten Benetzungseffekten und Schaumarmut kombinieren.

## Patentansprüche (Patentansprüche für folgende(n) Vertragsstaat(en): BE, DE, FR, GB, IT, NL, SE)

1. Alkandiolbisacetale der allgemeinen Formel I 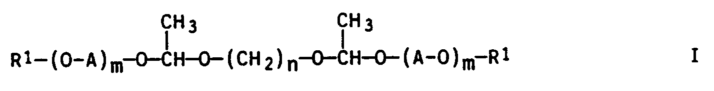 in der die Variablen
R¹ Alkyl- oder Alkenylgruppen mit 6 bis 30 C-Atomen bedeuten,
A 1,2-Alkylengruppen mit 2 bis 4 C-Atomen bezeichnen und
m für eine Zahl von 0 bis 50 stehen,
wobei die Variablen R¹, A und m jeweils gleich oder verschieden sein können, und
n eine Zahl von 2 bis 20 bedeutet.

2. Alkandiolbisacetale I nach Anspruch 1, bei denen die Variablen R¹ Alkyl- oder Alkenylgruppen mit 9 bis 18 C-Atomen bedeuten.

3. Alkandiolbisacetale I nach Anspruch 1 oder 2, bei denen die Variablen A Ethylen bezeichnen.

4. Alkandiolbisacetale I nach den Ansprüchen 1 bis 3, bei denen die Variablen m für eine Zahl von 2 bis 15 stehen.

5. Alkandiolbisacetale I nach den Ansprüchen 1 bis 4, bei denen die Variable n eine Zahl von 2 bis 10 bedeutet.

6. Verfahren zur Herstellung von Alkandiolbisacetalen I gemäß den Ansprüchen 1 bis 5, dadurch gekennzeichnet, daß man Alkandiolbisvinylether der allgemeinen Formel II
H₂C=CH-O-(CH₂)ₙ-O-CH=CH₂ II
in der n die oben genannte Bedeutung hat, mit alkoxylierten Alkoholen der allgemeinen Formel III
R¹-(O-A)ₘ-OH III
in der R¹, A und m die oben genannten Bedeutungen haben, oder einem Gemisch solcher Alkohole bei Temperaturen von 0 bis 100°C in Gegenwart katalytischer Mengen Säure umsetzt.

7. Verwendung von Alkandiolbisacetalen I gemäß den Ansprüchen 1 bis 5 als oberflächenaktive Substanzen in Wasch- und Reinigungsmitteln.

8. Verwendung von Alkandiolbisacetalen I nach Anspruch 7 bei maschinell ablaufenden Reinigungsprozessen.

9. Wasch- und Reinigungsmittel, enthaltend neben den hierfür üblichen Bestandteilen 0,1 bis 50 Gew.-%, bezogen auf die Gesamtmenge der Zubereitung, eines oder mehrerer Alkandiolbisacetale I gemäß den Ansprüchen 1 bis 5.

10. Verfahren zum Waschen und Reinigen, dadurch gekennzeichnet, daß man hierbei Alkandiolbisacetale I gemäß den Ansprüchen 1 bis 5 als oberflächenaktive Substanzen verwendet.

## Patentansprüche (Patentansprüche für folgende(n) Vertragsstaat(en): ES)

1. Verfahren zur Herstellung von Alkandiolbisacetalen der allgemeinen Formel I 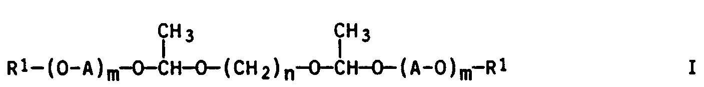 in der die Variablen
R¹ Alkyl- oder Alkenylgruppen mit 6 bis 30 C-Atomen bedeuten,
A 1,2-Alkylengruppen mit 2 bis 4 C-Atomen bezeichnen und
m für eine Zahl von 0 bis 50 stehen,
wobei die Variablen R¹, A und m jeweils gleich oder verschieden sein können, und
n eine Zahl von 2 bis 20 bedeutet, dadurch gekennzeichnet, daß man Alkandiolbisvinylether der allgemeinen Formel II
H₂C=CH-O-(CH₂)ₙ-O-CH=CH₂ II
in der n die oben genannte Bedeutung hat, mit alkoxylierten Alkoholen der allgemeinen Formel III
R¹-(O-A)ₘ-OH III
in der R¹, A und m die oben genannten Bedeutungen haben, oder einem Gemisch solcher Alkohole bei Temperaturen von 0 bis 100°C in Gegenwart katalytischer Mengen Säure umsetzt.

2. Verfahren zur Herstellung von Alkandiolbisacetalen I nach Anspruch 1, bei denen die Variablen R¹ Alkyl- oder Alkenylgruppen mit 9 bis 18 C-Atomen bedeuten.

3. Verfahren zur Herstellung von Alkandiolbisacetalen I nach Anspruch 1 oder 2, bei denen die Variablen A Ethylen bezeichnen.

4. Verfahren zur Herstellung von Alkandiolbisacetalen I nach den Ansprüchen 1 bis 3, bei denen die Variablen m für eine Zahl von 2 bis 15 stehen.

5. Verfahren zur Herstellung von Alkandiolbisacetalen I nach den Ansprüchen 1 bis 4, bei denen die Variable n eine Zahl von 2 bis 10 bedeutet.

6. Verfahren zur Herstellung von Wasch- und Reinigungsmitteln, dadurch gekennzeichnet, daß man hierfür übliche Bestandteile mit 0,1 bis 50 Gew.-%, bezogen auf die Gesamtmenge der Zubereitung, eines oder mehrerer Alkandiolbisacetale I gemäß den Ansprüchen 1 bis 5 mischt.

7. Verfahren zum Waschen und Reinigen, dadurch gekennzeichnet, daß man hierbei Alkandiolbisacetale I gemäß den Ansprüchen 1 bis 5 als oberflächenaktive Substanzen verwendet.

8. Verfahren zum Waschen und Reinigen mittels maschinell ablaufender Reinigungprozesse, dadurch gekennzeichnet, daß man hierbei Alkandiolbisacetale I gemäß den Ansprüchen 1 bis 5 als oberflächenaktive Substanzen verwendet.

## Claims (Claims for the following Contracting State(s): BE, DE, FR, GB, IT, NL, SE)

1. An alkanediol bisacetal of the general formula I 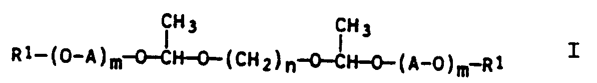 where
R¹ is alkyl or alkenyl of from 6 to 30 carbon atoms,
A is 1,2-alkylene of from 2 to 4 carbon atoms,
m is from 0 to 50,
the variables R¹, A and m being separately identical or different, and
n is from 2 to 20.

2. An alkanediol bisacetal I as claimed in claim 1, wherein R¹ is alkyl or alkenyl of from 9 to 18 carbon atoms.

3. An alkanediol bisacetal I as claimed in claim 1 or 2, wherein A is ethylene.

4. An alkanediol bisacetal I as claimed in any of claims 1 to 3, wherein m is from 2 to 15.

5. An alkanediol bisacetal I as claimed in any of claims 1 to 4, wherein n is from 2 to 10.

6. A process for preparing an alkanediol bisacetal I as claimed in any of claims 1 to 5, which comprises reacting an alkanediol bisvinyl ether of the general formula II
H₂C=CH-O-(CH₂)ₙ-O-CH=CH₂ II
where n is as defined above, with an alkoxylated alcohol of the general formula III
R¹-(O-A)ₘ-OH III
where R¹, A and m are each as defined above, or with a mixture of such alcohols at from 0 to 100°C in the presence of a catalytic amount of an acid.

7. The use of an alkanediol bisacetal I as claimed in any of claims 1 to 5 as surface-active substance in washing or cleaning agents.

8. A use as claimed in claim 7 in mechanized cleaning processes.

9. A washing or cleaning agent, containing from 0.1 to 50% by weight, based on the total amount of the formulation, of one or more alkanediol bisacetals I as claimed in any of claims 1 to 5 as well as customary constituents.

10. A method of washing or cleaning, which comprises using an alkanediol bisacetal I as claimed in any of claims 1 to 5 as surface-active substance.

## Claims (Claims for the following Contracting State(s): ES)

1. A process for preparing an alkanediol bisacetal of the general formula I 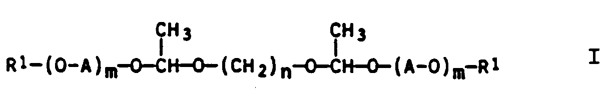 where
R¹ is alkyl or alkenyl of from 6 to 30 carbon atoms,
A is 1,2-alkylene of from 2 to 4 carbon atoms,
m is from 0 to 50,
the variables R¹, A and m being separately identical or different, and
n is from 2 to 20, which comprises reacting an alkanediol bisvinyl ether of the general formula II
H₂C=CH-O-(CH₂)ₙ-O-CH=CH₂ II
where n is as defined above, with an alkoxylated alcohol of the general formula III
R¹-(O-A)ₘ-OH III
where R¹, A and m are each as defined above, or with a mixture of such alcohols at from 0 to 100°C in the presence of a catalytic amount of an acid

2. A process for preparing an alkanediol bisacetal I as claimed in claim 1, wherein R¹ is alkyl or alkenyl of from 9 to 18 carbon atoms.

3. A process for preparing an alkanediol bisacetal I as claimed in claim 1 or 2, wherein A is ethylene.

4. A process for preparing an alkanediol bisacetal I as claimed in any of claims 1 to 3, wherein m is from 2 to 15.

5. A process for preparing an alkanediol bisacetal I as claimed in any of claims 1 to 4, wherein n is from 2 to 10.

6. A process for producing a washing or cleaning agent, which comprises mixing customary constituents with from 0.1 to 50% by weight, based on the total amount of the formulation, of one or more alkanediol bisacetals I as claimed in any of claims 1 to 5.

7. A method of washing or cleaning, which comprises using an alkanediol bisacetal I as claimed in any of claims 1 to 5 as surface-active substance.

8. A method of washing and cleaning by means of a mechanized cleaning process, which comprises using an alkanediol bisacetal I as claimed in any of claims 1 to 5 as surface-active substance.

## Revendications (Revendications pour l'(les) Etat(s) contractant(s) suivant(s): BE, DE, FR, GB, IT, NL, SE)

1. Alcanediolbisacétals de formule générale I 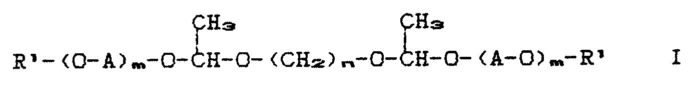 dans laquelle les variables ont les significations suivantes:
R¹ Groupements alkyle ou alcényle à 6-30 atomes de carbone,
A Groupements 1,2-alkylène à 2-4 atomes de carbone,
m Nombre de 0 à 50,
les variables R¹, A et m pouvant chaque fois être identiques ou différentes, et
n est un nombre de 2 à 20.

2. Alcanediolbisacétals I selon la revendication 1, dans lesquels les variables R¹ représentent des groupements alkyle ou alcényle à 9-18 atomes de carbone.

3. Alcanediolbisacétals I selon la revendication 1 ou 2, dans lesquels les variables A représentent des restes éthyléne.

4. Alcanediolbisacétals I selon l'une quelconque des revendications 1 à 3, dans lesquels les variables m sont mises pour un nombre de 2 à 15.

5. Alcanediolbisacétals I selon l'une quelconque des revendications 1 à 4, dans lesquels la variable n est mise pour un nombre de 2 à 10.

6. Procédé de préparation d'alcanediolbisacétals I selon l'une quelconque des revendications 1 à 5, caractérisé en ce qu'on fait réagir des éthers bisvinyliques d'alcanediol de formule générale II
H₂C=CH-O-(CH₂)ₙ-O-CH=CH₂ II
dans laquelle n a la siginication indiquée ci-dessus, avec des alcools alcoxylés de formule générale III
R¹-(O-A)ₘ-OH III
dans laquelle R¹, A et m ont les significations indiquées ci-dessus, ou avec un mélange de tels alcools, à des températures de 0 à 100°C, en présence de quantités catalytiques d'acide.

7. Utilisation d'alcanediolbisacétals I selon l'une quelconque des revendications 1 à 5 comme substances tensio-actives dans des produits de lavage et de nettoyage.

8. Utilisation d'alcanediolbisacétals I selon la revendication 7 dans des opérations de nettoyage effectuées à la machine.

9. Produits de lavage et de nettoyage contenant, outre les constituants usuels à cet effet, de 0,1 à 50% en poids, par rapport à la quantité totale de la préparation, d'un ou de plusieurs alcanediolbisacétals I selon l'une quelconque des revendications 1 à 5.

10. Procédé de lavage et de nettoyage, caractérisé en ce qu'on y utilise, comme substances tensio-actives, des alcanediolbisacétals I selon l'une quelconque des revendications 1 a 5.

## Revendications (Revendications pour l'(les) Etat(s) contractant(s) suivant(s): ES)

1. Procédé de préparation d'alcanediolbisacétals de formule générale I 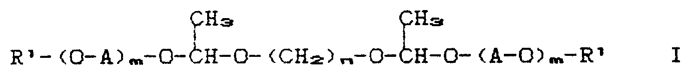 dans laquelle les variables ont les significations suivantes:
R¹ Groupements alkyle ou alcényle à 6-30 atomes de carbone,
A Groupements 1,2-alkylène à 2-4 atomes de carbone,
m Nombre de 0 à 50,
les variables R¹, A et m pouvant chaque fois être identiques ou différentes, et
n est un nombre de 2 à 20,
caractérisé en ce qu'on fait réagir des éthers bisvinyliques d'alcanediol de formule générale II
H₂C=CH-O-(CH₂)ₙ-O-CH=CH₂ II
dans laquelle n a la signification indiquée ci-dessus, avec des alcools alcoxylés de formule générale III
R¹-(O-A)ₘ-OH III
dans laquelle R¹, A et m ont les significations indiquées ci-dessus, ou avec un mélange de tels alcools, à des températures de 0 à 100°C en présence de quantités catalytiques d'acide.

2. Procédé selon la revendication 1 pour la préparation d'alcanediolbisacétals I dans lesquels les variables R¹ représentent des groupements alkyle ou alcényle à 9-18 atomes de carbone.

3. Procédé selon la revendication 1 ou 2 pour la préparation d'alcanediolbisacétals I dans lesquels les variables A représentent des restes éthylène.

4. Procédé selon l'une quelconque des revendications 1 à 3 pour la préparation d'alcanediolbisacétals I dans lesquels les variables m sont mises pour un nombre de 2 à 15.

5. Procédé selon l'une quelconque des revendications 1 à 4 pour la préparation d'alcanediolbisacétals I dans lesquels la variable n est mise pour un nombre de 2 à 10.

6. Procède de fabrication de produits de lavage et de nettoyage, caractérisé en ce qu'on mélange des constituants usuels à cet effet avec 0,1 à 50% en poids, par rapport à la quantité totale de la préparation, d'un ou de plusieurs alcanediolbisacétals I selon l'une quelconque des revendications 1 à 5.

7. Procède de lavage et de nettoyage, caractérisé en ce qu'on y utilise, comme substances tensio-actives, des alcanediolbisacétals I selon l'une quelconque des revendications 1 à 5.

8. Procédé de lavage et de nettoyage par des opérations de nettoyage effectuées à la machine, caractérise en ce qu'on y utilise, comme substances tensio-actives, des alcanediolbisacétals I selon l'une quelconque des revendications 1 à 5.
